# EUROPEAN PATENT APPLICATION

(11) **EP 2 322 147 A1**
(43) Date of publication of application: **18.05.2011**
(21) Application number: 09800099.5
(22) Date of filing: 23.07.2009
(51) Int. Cl.: A61K 9/62, C07K 14/475, C07K 14/515, A61P 25/14, A61P 25/16, A61P 25/28

(54) **USE OF MICROPARTICLES CONTAINING GENETICALLY MODIFIED CELLS IN THE TREATMENT OF NEURODEGENERATIVE DISEASES**

(30) Priority: 24.07.2008 ES 200802217
(71) Applicant: Universidad del Pais Vasco, 48940 Leioa Vizcaya (ES); Fundación Investigación Biomédica Hospital Universitario 12 Octubre, 28041 Madrid (ES); Centro De Investigación Biomédica En Red De Enfermedades Neurodegenerativas, 41013 Sevilla (ES)
(72) Inventor: HERNÁNDEZ MARTÍN, Rosa María, E-01006 Vitoria (ES); ORIVE ARROYO, Gorka, E-01006 Vitoria (ES); SPUCH CALVAR, Carlos, E-28031 Madrid (ES); ANTEQUERA TIENDA, Desiree, E-28041 Madrid (ES); BERMEJO-PAREJA, Félix, E-28041 Madrid (ES); CARRO DÍAZ, Eva, E-28041 Madrid (ES); PEDRAZ MUÑOZ, José Luis, E-01006 Vitoria (ES)
(74) Representative: ABG Patentes, S.L.
(86) International application number: PCT/ES2009/070309
(87) International publication number: WO 2010/010223

(57) **Abstract**

The present invention relates to methods for the treatment of neurodegenerative diseases by means of the use of microparticles comprising genetically modified cells expressing neurotrophic and/or angiogenic factors and wherein said microparticles are administered by means of implantation at the cerebral cortex level.

## Description

### Field of the Invention

The present invention relates to the use of microparticles comprising genetically modified cells expressing at least one neurotrophic factor, at least one angiogenic factor or a combination of both for the treatment of neurodegenerative diseases.

### Background of the Invention

Neurodegenerative diseases, including Alzheimer's disease (AD) and Parkinson's disease (PD) among others, are a serious problem from the medical, healthcare, social and economic point of view which developed countries must deal with.

There are currently several medicaments which provide a symptomatic treatment of these diseases, but none has proved to be useful with clinical relevance to slow down the progression of the degenerative process.

Angiogenic factors are growth factors which not only stimulate neovascularization and angiogenesis (initiated with the activation of endothelial cells of parent blood vessels) *in vivo,* but are also mitogenic for endothelial cells *in vitro*. Examples of angiogenic factors are, for example, HGF, VEGF, FGF and HIF. VEGF (vascular endothelial growth factor) is the prototype of angiogenic factors, the role of which at the central nervous system (CNS) level is not only related to the growth of blood vessels, as a physiological regulator of cerebral angiogenesis and of the integrity of the blood-brain barrier, but rather it also has a direct effect on different types of neural cells, even including neural stem cells (NSCs). In addition, conducted studies show that in diseases such as AD or Huntington's disease there are cerebrovascular deficiencies which precede the onset of the clinical symptoms, which suggests that said alterations could contribute to the pathogenesis of these diseases.

Neurotrophic factors are natural proteins which play an important role at the CNS level. Said factors are essential to assure the survival and the differentiation of neurons during development and to maintain normal neuronal function in adults. Due to these physiological functions, neurotrophic factors are useful for the treatment of CNS pathologies in which the survival and/or the neuronal function itself are compromised. Neurotrophic factors include GDNF (glial cell-derived neurotrophic factor) which was isolated by Lin *et al.* in 1930 and its use in different diseases affecting the CNS has been assayed since then.

An important problem to be solved is the system and the site of administration of these angiogenic and neurotrophic factors, since it is necessary for the factor to cross the blood-brain barrier so that it can exert its action and, furthermore, the treatment of these diseases can entail a chronic administration for long time periods.

Based on prior knowledge, there is a large amount of therapeutic products, such as the neurotrophic and angiogenic factors mentioned above, for example, for the treatment of CNS diseases. Nevertheless, the release of said products at the brain level is limited by the difficulty involved in the access to the site of administration. In clinical practice, the controlled release of these therapeutic products is performed by means of infusion pumps or cannulas at the intraventricular level. These administration routes require continuous injections or reloads of the infusion pump to maintain the levels of the drug and to prevent the degradation of the therapeutic substance. Furthermore, due to the technology of current infusion pumps it is difficult to administer low doses of drugs for prolonged timed periods.

One of the therapeutic strategies which is acquiring more importance in the treatment of neurodegenerative and CNS diseases is cell therapy. The use of cells as a medicament is a therapeutic alternative of increasing interest in the scientific community, with which the intention is to develop pharmaceutical systems which release the drug secreted by the cells for long time periods and in a safe and physiological manner, being especially suitable for the treatment of chronic diseases. Nevertheless, one of the main limitations of this type of therapy is the rejection of the implanted cells by the immune response of the host, provided that the graft object of the transplant does not come from the actual host. This is why the administration of allogeneic and xenogeneic cells which secrete therapeutic products offer unsatisfactory mid-long term results as a consequence of the action of the defense mechanisms of the patient. Due to this drawback, systems have been designed which allow the administration of cells and which prevent the rejection caused by the immune response, hollow fibers and microcapsules stand out among said systems.

The use of hollow fibers containing cells which are genetically modified to produce CNTF (Ciliary Neurotrophic Factor) is a system of administration which has been used in the treatment of patients with ALS. Likewise, processes comprising the encapsulation of GDNF or VEGF-producing cells in hollow fibers which are implanted at the striatum level have also been described (Yasuhara, T. and Date, I. 2007. Cell transplantation, vol. 16:1-8; Lindvall, O. and Wahlberg, L.U. 2008, Experimental Neurology, vol. 209:82-88). European patent application EP0388428 describes methods in which ventral mesencephalon fragments encapsulated in tubes formed by semipermeable membranes are implanted in the parietal region of the cerebral cortex after a craniotomy. Nevertheless, the drawback of this type of technology is that it is a system with a large size (of the order of millimeters), which can condition the viability and functionality of the encapsulated cells.

An alternative to the hollow fibers is the use of microcapsules which can comprise choroid plexus cells or fragments, or cells which are genetically modified to express proteins of therapeutic interest.

Skinner, S.M.J. et al. (Xenotransplantation, 2006 vol. 13: 284-288) describe the use of microencapsulated choroid plexus cells in animal models of different CNS diseases.

Borlongan et al. (Neurochemistry Int. 2004 vol. 24: 495-503) describe a method for the treatment of cerebral ischemia by means of the subdural implantation of microcapsules comprising choroid plexus cells.

United States patent application US2004213768 describes methods for the administration of neurotrophic factors to the central nervous system based on the implantation of microcapsules (preferably alginate microcapsules) comprising isolated choroid plexus cells, wherein the implantation is performed in a subdural or subarachnoid manner.

However, the use of choroid plexus cells does not allow accurately controlling the nature of the angiogenic/neurotrophic factors produced by the cell.

In addition, in relation to the use of microcapsules which can comprise genetically modified cells, Meysinger, D. et al. (Neurochem. Int. 1994 vol. 24(5): 495-503) describe alginate-polylysine-alginate microcapsules comprising fibroblasts which are genetically modified to produce nerve growth factor (NGF). A work published by Grandoso, L. et al. (Internal Journal of Pharmaceutics, 2007 vol. 343:69-78) studies the use of alginate-poly-L-lysine microcapsules in which GDNF-producing fibroblasts are immobilized for the treatment of PD in a parkinsonian rat model. The microcapsules were administered at the striatum level by means of stereotaxis and an improvement was observed in the rotational behavior of the animals.

However, both works use extremely invasive techniques which can lead to undesirable side effects in the patient.

There is therefore a need in the state of the art to find systems which allow controlling the therapeutic product and/or the angiogenic and neurotrophic factors administered and which are less invasive, thus improving the treatment of neurodegenerative diseases, among which AD and PD are included.

### Summary of the Invention

In one aspect, the invention relates to a microparticle comprising genetically modified cells expressing at least one neurotrophic factor, at least one angiogenic factor or a combination of both for the treatment of neurodegenerative diseases, wherein the microparticle is administered at the cerebral cortex level.

### Brief Description of the Drawings

Figure 1 shows three photographs of the microparticles containing fibroblasts which are genetically modified to produce VEGF. A and B: optical microscopy images. C: fluorescence microscopy image.
Figure 2 is a graph showing the proliferation of BMVEC cells in the presence of VEGF secreted by the microparticles (33 ng/mL) and of the VEGF (500 ng/mL) stock solution at days 7 and 20.
Figure 3 is a graph showing the blood vessel density stimulated by the production of VEGF from the microparticles implanted in normal C57BL/6 mice.
Figure 4 is a photograph showing the double BrdUrd-lectin staining of the new blood vessels induced by the production of VEGF from the microparticles implanted in normal C57BL/6 mice.
Figure 5 is a graph depicting the results obtained in the T-maze test in mice APP/PS1.
Figure 6 is a graph showing the results of the object recognition test in APP/PS1 mice.
Figure 7 is a graph depicting the levels of BDNF measured in different tissues of control (treated with empty microparticles) old rats (18-24 months) and rats treated with microparticles which contained BDNF-producing cells.
Figure 8 is a graph depicting the results of the determination of caspase 3, caspase 9 and PMAPK in the cortex of control old rats (treated with empty microparticles) and with the microparticles releasing BDNF.

### Detailed Description of the Invention

The inventors have discovered that the administration at the cerebral cortex level of microparticles comprising cells which are genetically modified to express neurotrophic or angiogenic factors surprisingly allows the treatment of not only lesions located in the cerebral cortex but also of lesions located in the other areas of the brain as a result of the diffusion of the neurotrophic or angiogenic factors administered.

Thus, in one aspect, the invention relates to a microparticle comprising genetically modified cells expressing at least one neurotrophic factor, at least one angiogenic factor or a combination of both, for the treatment of neurodegenerative diseases, wherein the microparticle is administered at the cerebral cortex level.

The microparticle of the invention is a spherical or non-spherical particle, inside which there is included (i) microcapsules, which are defined as vesicular systems in which the genetically modified cells are confined in a cavity surrounded by a single (usually polymeric) membrane; and (ii) microspheres, which are matrix systems in which the cells are dispersed over the entire particle.

In the present invention, "microparticle" is understood as that particle comprising a diameter less than 1 mm, preferably between 1 and 0.9, between 0.9 and 0.8, between 0.8 and 0.7, between 0.7 and 0.6, between 0.6 and 0.5, between 0.5 and 0.4, between 0.4 and 0.3, between 0.3 and 0.2, between 0.2 and 0.1 or less than 0.1 mm in diameter. In a particular embodiment, the microparticle of the invention has a diameter between 0.380 and 0.404 mm, preferably, 0.392 mm. Likewise, in the context of the present invention and due to the capacity of the microparticle to continuously produce therapeutic products, said microparticle is referred to as a pharmaceutical microbiosystem.

Nevertheless, as will be understood by the person skilled in the art, the average size of the microparticle of the invention is affected by different technological factors of the process for producing said microparticle, such as the concentration of the different components of the microparticle, stirring speed, etc.

The microparticle of the invention can be formed by any biocompatible polymeric material allowing the continuous secretion of the therapeutic products and acting as a support of the genetically modified cells. Thus, said biocompatible polymeric material can be, for example, thermoplastic polymers or hydrogel polymers.

The thermoplastic polymers include acrylic acid, acrylamide, 2-aminoethyl methacrylate, poly(tetrafluoroethylene-co-hexafluoropropylene), methacrylic acid-(7-coumaroxy)ethyl ester, N-isopropylacrylamde, polyacrylic acid, polyacrylamide, polyamidoamine, poly(amino)-p-xylylene, poly(chloroethyl vinyl ether), polycaprolactone, poly(caprolactone-co-trimethylene carbonate), poly (carbonate-urea)urethane, poly(carbonate)urethane, polyethylene, polyethylene and acrylamide copolymer, polyethylene glycol, polyethylene glycol methacrylate, poly(ethylene terephthalate), poly(4-hydroxybutyl acrylate), poly(hydroxyethyl methacrylate), poly(N-2-hydroxypropyl methacrylate), poly(lactic acid-glycolic acid), poly(L-lactic acid), poly(gamma-methyl, L-glutamate), poly(methylmethacrylate), poly(propylene fumarate), poly(propylene oxide), polypyrrole, polystyrene, poly(tetrafluoroethylene), polyurethane, polyvinyl alcohol, ultra high molecular weight polyethylene, 6-(p-vinylbenzamido)-hexanoic acid and N-p-vinylbenzyl-D-maltonamide and copolymers containing more than one of said polymers.

The hydrogel type polymers include natural materials of the type of alginate, agarose, collagen, starch, hyaluronic acid, bovine serum albumin, cellulose and derivatives thereof, pectin, chondroitin sulfate, fibrin and fibroin, as well as synthetic hydrogels such as sepharose and sephadex.

In a particular embodiment, the polymer forming part of the microparticle of the invention is bound to, or functionalized with, a ligand specific for a cell surface receptor. In the present invention, "ligand specific for a cell surface receptor" is understood as the molecule or peptide which is capable of recognizing a cell surface receptor and binding to it in a specific manner. Thus, said ligand allows the specific interaction between the polymer of the microparticle and the genetically modified cells contained therein.

In principle, any molecule or peptide having specific binding sites which can be recognized by cell surface receptors can be used in the present invention as a ligand specific for a cell surface receptor. Thus, the ligand specific for a cell surface receptor can come from cell adhesion molecules which interact with the extracellular matrix such as fibronectin, the different members of the families of selectins, cadherins, lectins, integrins, immunoglobulins, collectins and galectins.

Thus, the ligand specific for a cell surface receptor used in the present invention can be a peptide derived from a region selected from the regions of fibronectin which are involved in the binding with integrins which are located in the cell membrane. For example, and without limiting the invention, said peptides are derived from the region of the tenth type III repeat of fibronectin containing the RGD peptide, from the region of the fourteenth type III repeat of fibronectin containing the IDAPS peptide (SEQ ID NO: 1), from the CSI region of fibronectin containing the LDV peptide and the CS5 region of fibronectin containing the REDV peptide (SEQ ID NO: 2). These peptides can consist of fragments of the corresponding regions which conserve their adhesive capacity, such as the QAGDV peptide (SEQ ID NO: 3) of fibrinogen, the LDV peptide of fibronectin and the IDSP peptide (SEQ ID NO: 4) of VCAM-I, for example.

The present invention also contemplates the use of integrin-binding peptides as a ligand specific for a cell surface receptor, which are derived from the region of the tenth type III repeat of fibronectin comprising the RGD sequence, such as, for example, a peptide selected from the group of RGD, RGDS (SEQ ID NO: 5), GRGD (SEQ ID NO: 6), RGDV (SEQ ID NO: 7), RGDT (SEQ ID NO: 8), GRGDG (SEQ ID NO: 9), GRGDS (SEQ ID NO: 10), GRGDY (SEQ ID NO: 11), GRGDF (SEQ ID NO: 12), YRGDS (SEQ ID NO: 13), YRGDDG (SEQ ID NO: 14), GRGDSP (SEQ ID NO: 15), GRGDSG (SEQ ID NO: 16), GRGDSY (SEQ ID NO: 17), GRGDVY (SEQ ID NO: 18), GRGDSPK (SEQ ID NO: 19), CGRGDSPK (SEQ ID NO: 20), CGRGDSPK (SEQ ID NO: 21), CGRGDSY (SEQ ID NO: 22), cyclo (RGDfK) (SEQ ID NO: 23), YAVTGRGD (SEQ ID NO: 24), AcCGGNGEPRGDYRAY-NH2 (SEQ ID NO: 25), AcGCGYGRGDSPG (SEQ ID NO: 26) and RGDPASSKP (SEQ ID NO: 27), cyclic variants of said peptides, both linear and branched multivalent variants (see for example Dettin et al. 2002, J. Biomed. Mater. Res. 60:466-471; Monaghan et al. 2001, Arkivoc, 2: U42-49; Thumshirn et al. 2003, Chemistry 9: 2717-2725; Scott et al, 2001, J. Gene Med. 3: 125-134) as well as combinations of two or more of said peptides.

Therefore, in another particular embodiment, the ligand specific for a cell surface receptor is a peptide comprising the RGD sequence.

The peptide comprising the RGD sequence can be bound to the polymer of the microparticle through the N-terminal end or through the C-terminal end and, independently of the anchoring point, can be bound directly to the polymer or, alternatively, can be bound through a spacer element. Virtually any peptide with structural flexibility can be used. By way of illustration, said flexible peptide can contain repeats of amino acid residues, such as (Gly)₄ (SEQ ID NO: 28), Gly-Gly-Gly-Ser (SEQ ID NO: 29), (Gly)₁₃ (SEQ ID NO: 30) (Beer, J.H. et al., 1992, Blood, 79, 117-128), SGGTSGSTSGTGST (SEQ ID NO: 31), AGSSTGSSTGPGSTT (SEQ ID NO: 32), GGSGGAP (SEQ ID NO: 33), GGGVEGGG (SEQ ID NO: 34) or any other suitable repeat of amino acid residues, or the hinge region of an antibody.

Likewise, the ligand specific for a cell surface receptor can be bound to the polymer with different degrees of substitution, such that the concentrations of both components can vary and thus the number of ligands specific for a cell surface receptor which are bound to the polymer of the microparticle can be controlled. Thus, the invention contemplates polymers containing between 1 and 100, between 100 and 200, between 200 and 300, between 300 and 400, between 400 and 500, between 500 and 600, between 600 and 700, between 700 and 800, between 800 and 900 and between 900 and 1000 molecules of said specific ligand for each molecule of polymer.

As has been indicated above, the microparticle of the invention can be formed by any biocompatible polymeric material allowing the continuous secretion of the therapeutic products and acting as a support of the genetically modified cells. Thus, in a particular embodiment, the polymer of the microparticle of the invention is alginate. Example 1 of the present patent application describes one of the different processes existing in the state of the art for producing microparticles comprising alginate as a biopolymeric material.

In principle, any type of alginate capable of forming a hydrogel is suitable for being used in the microparticle of the invention. Thus, the microparticle can contain alginate mostly formed by regions of mannuronic acid (MM blocks), by regions of guluronic acid (GG blocks) and by mixed sequence regions (MG blocks). The percentage and distribution of the uronic acids differ according to the origin of the alginate and contribute to the properties of the alginate. The person skilled in the art knows the percentages of each of the different blocks appearing in the different biological sources of the alginates. Thus, the invention contemplates the use of alginates coming from *Laminaria hyperborea, Lessonia nigrescens, Lessonia trabeculata, Durvillaea antarctica, Laminaria digitata, Ecklonia maxima, Macrocystis pyrifera, Ascophyllum nodosum* and/or *Laminaria japonica* as well as mixtures of alginates of different species until achieving the desired content of GG, MM or GM blocks. The GG blocks contribute to the rigidity of the hydrogel, whereas the MM monomers maintain a high fracture resistance, such that by means of using a suitable combination of alginate polymers a mixture can be obtained the modulus of elasticity of which has a suitable value, whereas the viscosity of the pre-gel solution is maintained at levels low enough to allow a suitable cell handling and immobilization. Thus, the alginates which can be used in the present invention include GG alginates, MM alginates or combinations of both in a ratio of 90:10, 80:20, 70:30, 60:40, 50:50, 40:60, 30:70, 20:80 or 10:90.

Additionally, the invention also contemplates the use of alginates derived from the treatment of natural alginates with enzymes which are capable of modifying the component blocks to give rise to alginates with improved properties. Thus, alginates resulting from treating alginates with C5-epimerases, which convert M blocks into G blocks, as well as with the enzyme AlgE4 of the bacteria *Azotobacter vinelandii* which is capable of converting the relatively rigid M blocks into MG blocks. Alternatively, the invention contemplates the use of alginates which have been modified by different physical treatments, particularly, gamma rays, irradiation with ultrasound or with ultraviolet light as has been described by Wasikiewicz, J.M. et al. (Radiation Physics and Chemistry, 2005, 73:287-295).

The microparticle of the invention comprising alginate as a biocompatible polymeric material can used as is. However, as is known from the state of the art, alginate is an unstable polymer tending to lose calcium and, therefore, lose its gel nature. Furthermore, the alginate particles are relatively porous, which can result in the antibodies being able to access their interior and damaging the cells. For these reasons, the microparticle of the invention can optionally be surrounded by a semipermeable membrane which confers stability to the particles and which forms a barrier impermeable to the antibodies.

Semipermeable membrane is understood as a membrane which allows the entrance of all those solutes necessary for cell viability and allowing the exit of the therapeutic proteins produced by the cells contained inside the microparticle, but which is substantially impermeable to the antibodies, such that the cells are protected from the immune response produced by the organism housing the microparticle.

Materials suitable for forming the semipermeable membrane are materials insoluble in biological fluids, preferably polyamino acids, such as for example poly-L-lysine, poly-L-ornithine, poly-L-arginine, poly-L-asparagine, poly-L-aspartic acid, polybenzyl-L-aspartate, poly-S-benzyl-L-cysteine, poly-gamma-benzyl-L-glutamate, poly-S-CBZ-L-cysteine, poly-ε-CBZ-D-lysine, poly-8-CBZ-DL-ornithine, poly-O-CBZ-L-serine, poly-O-CBZ-D-tyrosine, poly(y-ethyl-L-glutamate), poly-D-glutamic acid, polyglycine, poly-γ-N-hexyl L-glutamate, poly-L-histidine, poly(α,β-[N-(2-hydroxyethyl)-DL-aspartamide]), poly-L-hydroxyproline, poly(α,β-[N-(3-hydroxypropyl)-DL-aspartamide]), poly-L-isoleucine, poly-L-leucine, poly-D-lysine, poly-L-phenylalanine, poly-L-proline, poly-L-serine, poly-L-threonine, poly-DL-tryptophan, poly-D-tyrosine or a combination thereof. Preferably,

Therefore, in a particular embodiment, the microparticle of the invention furthermore comprises a poly-L-lysine membrane.

The membrane coating the microparticle is usually made of a polycationic material, which gives rise to the formation of a polyanion-polycation complex contributing to the stabilization of the alginate and to reducing the porosity of the microparticle and to forming an immunological barrier impermeable to antibodies. However, the positive charge of said membrane favors cell adhesion to the surface of the microparticle, which results in a lower biocompatibility thereof. To that end, if desired, the poly-L-lysine membrane surrounding the microparticle is in turn surrounded by a second membrane mostly formed by a material which inhibits cell adhesion, preferably alginate (see Example 1 of the present patent application).

Any eukaryotic cell which has been genetically modified to express at least one neurotrophic factor, at least one angiogenic factor or a combination of both can be used in the present invention, but mouse, rat, primate and human cells are the preferred cells. Thus, cells suitable for carrying out the invention are cardiomyocytes, endothelial cells, epithelial cells, lymphocytes (B and T cells), mastocytes, eosinophils, vascular intima cells, primary cultures of cells isolated from different organs, preferably of cells isolated from islets of Langerhans, hepatocytes, leukocytes, including mononuclear leukocytes, embryonic, mesenchymal, umbilical cord or adult (skin, lung, kidney and liver) stem cells, osteoclasts, chondrocytes and other connective tissue cells. Cells of established lines such as Jurkat T cells, NIH-3T3 cells, CHO cells, Cos cells, VERO cells, BHK cells, HeLa cells, COS cells, MDCK cells, 293 cells, 3T3 cells, C2C12 myoblasts and W138 cells are also suitable.

In principle, the number of cells which must form part of the microparticle is not essential for the invention provided that there is a number of cells sufficient for contributing to the formation of the lattice. Thus, the amount of cells for each mL of polymer solution is between 1 and 10 x10⁶, preferably between 2 and 9 x10⁶, more preferably between 3 and 8 x10⁶, still more preferably between 4 and 7 x10⁶ and still more preferably between 5 and 6 x 10⁶. The number of cells in the initial mixture is preferably 5; 3.75; 2.5 or 1.25 x10⁶ for each mL of polymer solution.

In the context of the present invention, the eukaryotic cell contained inside the microparticle of the invention can be modified with any neurotrophic factor, for example, and without being limited to, neurotrophins (NTs), such as NT-3, NT-4, NT-5 or NT-6; brain-derived neurotrophic factor (BDNF); ciliary neurotrophic factor (CNTF); insulin-like growth factor type 1 (IGF-1); insulin-like growth factor type 2 (IGF-2); nerve growth factor (NGF); neurturin (NTN); persephins; artemins, pleiotrophin (PTN), ephrins, netrins, semaphorins, slits, reelins, glial cell-derived neurotrophic factor (GDNF), conserved dopamine neurotrophic factor (CDNF), mesencephalic astrocyte-derived neurotrophic factor (MANF), etc.

Likewise, in the context of the present invention, the eukaryotic cell contained inside the microparticle of the invention can be modified with any angiogenic factor, for example, and without being limited to, angiopoietins (Ang), such as Ang-1, Ang-2, Ang-3 or Ang-4; basic fibroblast growth factor (bFGF/FGF2), such as fibroblast growth factor 20 (FGF20), for example; transforming growth factor beta (TGFβ), transforming growth factor alpha (TGFα), placental growth factor (PIGF); epidermal growth factor (EGF); vascular endothelial growth factor (VEGF), such as VEGF-A, VEGF-B or VEGF-C; platelet-derived growth factor (PDGF), such as PDGF-A and PDGF-B; vasoactive intestinal polypeptide (VIP); hepatocyte growth factor (HGF), cardiotrophins, bone morphogenetic proteins (BMPs), sonic hedgehog (SHH), etc.

As will be understood by the person skilled in the art, the genetic modification of the cells which will be encapsulated inside the microparticle of the invention can be carried out by means of any method of those known in the art. Thus, the gene or the vector containing the gene can be administered by means of electroporation, transfection using liposomes or polycationic proteins or using viral vectors, including adenoviral and retroviral vectors and also non-viral vectors.

Likewise, the nucleotide sequences encoding the neurotrophic factors or the angiogenic factors are associated to sequences regulating the expression of said factors. These sequences can be transcription regulatory sequences, such as constitutive or inducible promoters, enhancers, transcription terminators and translation regulatory sequences, such as non-translated sequences located 5' or 3' with respect to the encoding sequence.

Promoters suitable for the expression of the neurotrophic factors or angiogenic factors include, without necessarily being limited to, constitutive promoters such as derivatives of the genomes of eukaryotic viruses such as the polyomavirus, adenovirus, SV40, CMV, avian sarcoma virus, hepatitis B virus, the metallothionein gene promoter, the herpes simplex virus thymidine kinase gene promoter, LTR regions of retroviruses, the immunoglobulin gene promoter, the actin gene promoter, the EF-1 alpha gene promoter as well as inducible promoters in which the expression of the protein depends on the addition of a molecule or on an exogenous signal, such as the tetracycline system, the NFkappaB/UV light system, the Cre/Lox system and the heat shock gene promoter.

In addition, the neurotrophic factors or angiogenic factors expressed by the cells forming part of the microparticle of the invention can be expressed transiently or stably. In the event that the microparticle remains for a long time in the patient, it is preferable to use cells expressing said factors stably. Stable expression requires the transformation of the polynucleotide encoding the neurotrophic factors or angiogenic factors to be performed together with a polynucleotide encoding a protein which allows selecting transformed cells. Suitable selection systems are, without limitation, herpes virus thymidine kinase, hypoxanthine-guanine phosphoribosyltransferase, adenine phosphoribosyltransferase, genes encoding proteins which confer resistance to an antimetabolite such as dihydrofolate reductase, pyruvate transaminase, the gene of resistance to neomycin and to hygromycin.

As has been indicated at the beginning of the present description, the invention relates to a microparticle comprising genetically modified cells expressing at least one neurotrophic factor, at least one angiogenic factor or a combination of both, for the treatment of neurodegenerative diseases, wherein the microparticle is administered at the cerebral cortex level.

As will be understood by the person skilled in the art, any neurodegenerative disease which can be treated with neurotrophic factors, angiogenic factors or a combination of both, can likewise be treated with the microparticle of the invention.

In the present invention, "neurodegenerative disease" is understood as that disease which is distinguished by being the result of a progressive death of neurons in the nervous system, fundamentally in the brain, giving rise to the worsening of body activities, such as balance, movement, speech, breathing, cardiac function, etc. Examples of neurodegenerative diseases are, without being limited to, Alzheimer's disease, amyotrophic lateral sclerosis, Friedreich's ataxia, Huntington's disease, dementia with Lewy bodies, Parkinson's disease, spinal muscular atrophy, etc.

In a particular embodiment, the neurodegenerative disease is selected from Alzheimer's disease, Parkinson's disease, amyotrophic lateral sclerosis and Huntington's disease.

In the context of the present invention, the microparticle is administered at the cerebral cortex level, allowing the diffusion of the neurotrophic or angiogenic factors to other areas of the brain. In the context of the present invention, administration "at the cerebral cortex level" is understood as a method in which the administration is carried out by means of perforation of the cranium and one or several meninges but without damaging the cerebral cortex.

In the context of the present invention, the preferred method of administration is by means of a craniotomy.

In the present invention, "craniotomy" is understood as the brain surgery which consists of opening the cranium to expose the meninges, allowing the administration of the microparticles in a subdural or subarachnoid manner.

In a particular embodiment of the invention, the administration of the microparticle at the cerebral cortex level is carried out in a subdural or subarachnoid manner. The process for administering the microparticles is described in Example 2 attached to the present description.

In the present invention, "cerebral cortex" or "brain cortex" is understood as the layer of nervous tissue covering the surface of the cerebral hemispheres.

In a particular embodiment, the administration of the microparticle at the cerebral cortex level is carried out by means of a bilateral craniotomy.

In another aspect, the invention relates to a method for the treatment of neurodegenerative diseases by means of the administration of microparticles comprising genetically modified cells expressing at least one neurotrophic factor, at least one angiogenic factor or a combination of the above, wherein the microparticles are administered at the cerebral cortex level.

The following examples are illustrative of the invention and do not intend to limit same.

### Example 1

### Preparation and characterization of the microparticles Culture of the VEGF-producing cells

The BHK cells (fibroblasts from hamster kidney) which are genetically modified to produce VEGF were grown in DMEM medium with 2% L-glutamine, 10% fetal bovine serum (FBS) and 1% antibiotic/antimycotic. Passages of the cells were performed every 2 or 3 days, being maintained in the incubator at 37°C in a 5% CO₂ atmosphere. All the components of the culture media used were from the company Gibco BRL (Invitrogen S.A., Spain).

### Encapsulation of the cells in the microparticles

The encapsulation process comprises several steps. First, the cells, which are genetically modified to secrete the therapeutic product, are suspended in an alginate solution. The cell suspension is subsequently passed through the tip of the electrostatic droplet generator by means of a flow pump, the droplets formed falling into the gelling calcium chloride solution. Furthermore, by means of the application of an electrostatic potential difference between the tip of the droplet generator and the calcium chloride solution, the formation and gelling of small droplets from the cell suspension are achieved. Once the solid alginate cores are formed, they are coated, applying a first coating with a 0.05% poly-L-lysine solution for 5 minutes and a second coating with a 0.1% alginate solution for 5 minutes.

### Characterization of the microparticles

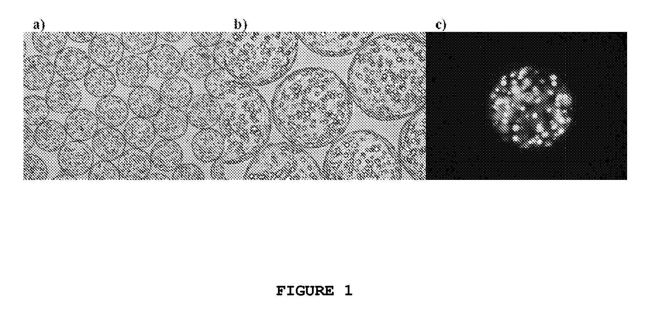

The size and the surface characteristics of the microparticles were determined by using an inverted optical microscope (NikonTSM) equipped with a camera (Sony CCD-Iris). The microparticles obtained have a spherical shape and a smooth and uniform surface the average size of which was 392 ± 12 µm (Figure 1).

### Viability and functionality of the VEGF-producing fibroblasts in the microparticles

To characterize the viability and functionality of the fibroblasts inside the microparticles, the cellular metabolic activity and the release of VEGF were studied *in vitro* for a period of three weeks. The cell viability was determined by using the MTT assay. The production of VEGF was determined by means of an ELISA technique (Amersham Biosciences, USA). After 21 days in culture, the secretion of VEGF from the microparticles loaded with 10⁶ cells per milliliter of alginate was approximately 174 ng of VEGF/24 hours. These results suggest that the cells adapted satisfactorily to the new microenvironment.

### In vitro proliferation assay

The BMVEC cell line are cells derived from brain endothelial cells. They are maintained in DMEM medium (Sigma), 10% FBS (Gibco) and 1% antibiotic-antimycotic (Gibco).

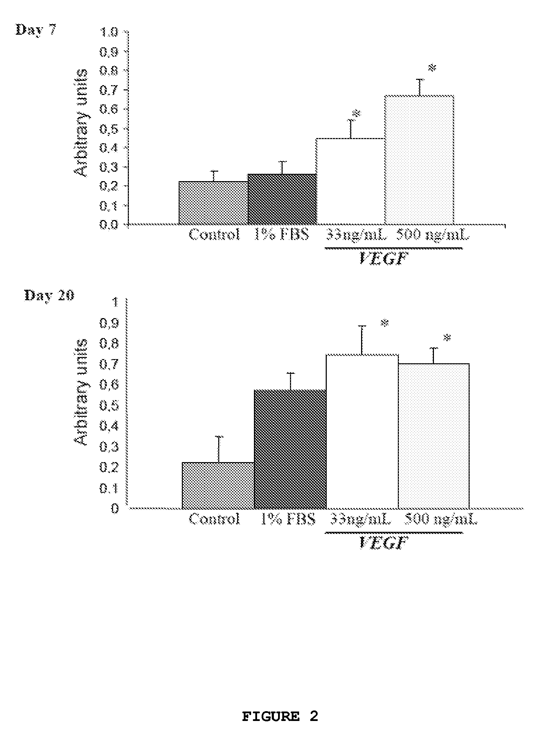

The brain endothelial cells, BMVECs, were cultured in the presence of different concentrations of VEGF prepared from the pure product, stock solution (500 ng/mL) and secreted from the microparticles, functional VEGF (33 ng/mL). The cell proliferation was determined by means of the XTT Proliferation Kit (Roche) at 7 and 20 days (Figure 2). This assay allows determining the functionality of the VEGF released from the microparticles. The concentration of 33 ng/mL is similar to that produced by about 200 microparticles and the effect thereof was compared with a high concentration of VEGF such as 500 ng/mL. The results obtained show that at 7 days both the treatment with the stock solution and with the functional VEGF stimulate cell proliferation and that the latter is significantly higher than that produced by the controls. Surprisingly, at 20 days it is observed that low concentrations of VEGF induce a higher cell proliferation than the high concentrations, which shows that the dose of VEGF secreted by the microparticles could be enough to induce the proliferative response without there being risks of onset of edema caused by an excessively high concentration of VEGF.

### Example 2

### Implantation of the microparticles in normal C57BL/6 mice and histological analysis

The animals were divided into two groups of 6 mice each: control group (which received empty microparticles) and group treated with VEGF (n=6) in which the microparticles which contained the VEGF-producing cells were implanted at the cerebral cortex level. The mice anesthetized by means of inhaled isoflurane were subjected to a bilateral craniotomy in the coordinates posterior 0.6 mm and lateral 1.1 mm in relation to the bregma point (Paxinos & Watson, 1986. "The rat brain in stereotaxic coordinates", 2nd edition, New York: Academia). Once the craniotomy was performed, the VEGF-producing microparticles were administered to them in the surface of the brain and empty microparticles were administered to the control mice. Between 20-30 microparticles per hole were administered to each mouse. Once the surgical intervention had ended, the holes were closed with nitrocellulose membranes impregnated in a diluted iodine (Betadine) solution, which prevents the exit of the capsules from the holes and isolates the brain from possible infection.

The animals were sacrificed at 2 weeks, 1 month and 3 months and immunohistochemistry studies were conducted. The formation of vessels was determined by using biotinylated tomato lectin and factor VIII.

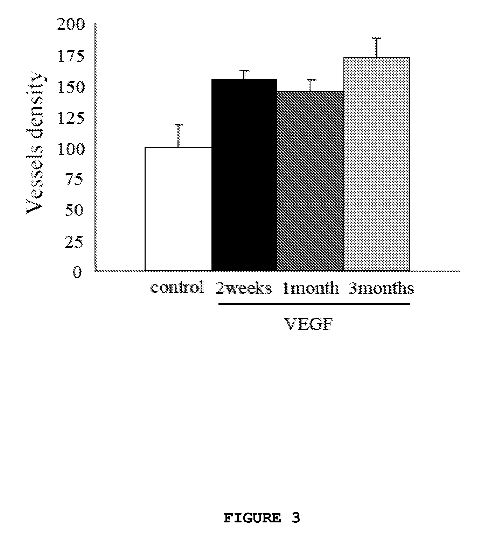

The results obtained showed that the density of the blood vessels in the brains of the animals treated with the microparticles which contained the VEGF-producing cells was clearly greater than that of the animals of the control group at two weeks, and that the effect was maintained at one month and at two months, which indicates a sustained release of the molecule from the microparticles (Figure 3).

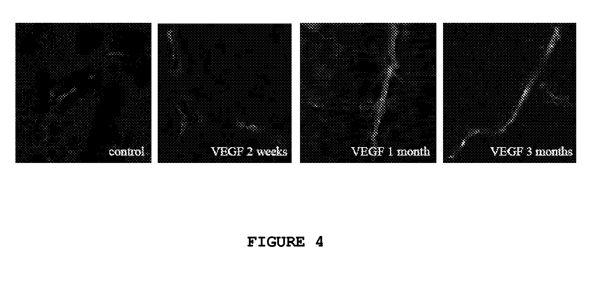

Furthermore, a double BrdUrd and lectin staining was performed for the purpose of distinguishing the new vessels formed after the release of VEGF from the pre-existing vessels, observing an increase of said vessels as the time since the administration goes by (Figure 4).

### EXAMPLE 3

### Treatment of transgenic animals with Alzheimer's disease with microparticles in which cells which are genetically modified to produce VEGF have been encapsulated

The double transgenic mice: Amyloid precursor protein/presenilin-1 (APP/Ps1) resulting from the cross between the Tg2576 mouse (overexpressing the human APP695 protein) and the mutant mouse (M146L mouse) for presenilin-1 (Ps1). These mice are an amyloidosis model for Alzheimer's disease.

The mice anesthetized by means of inhaled isoflurane were subjected to a bilateral craniotomy in the coordinates posterior 0.6 mm and lateral 1.1 mm in relation to the bregma point (Paxinos and Watson, 1982). Once the craniotomy was performed, VEGF-producing microparticles were administered to a group of mice in the surface of the brain at subdural level, and microparticles with non-transfected fibroblasts were administered to another group of mice (this group of mice was referred to as "sham"). A group of control littermate mice was furthermore included. Between 20-30 microparticles per hole were administered to each mouse (6 per group). Once the surgical intervention had ended, the holes were closed with nitrocellulose membranes impregnated in a diluted iodine (Betadine) solution, which prevents the exit of the capsules from the holes and isolates the brain from possible infection.

The mice were maintained for 3 months in the animal housing unit until they were subjected to the behavioral tests: T-Maze test and the object recognition test. When the behavioral tests ended, the mice were transcardially perfused with 0.9% saline solution. Half the brain was fixed with 4% paraformaldehyde in 0.1 M saline solution, pH 7.4, and the other half was frozen at -80°C for subsequent biochemical tests.

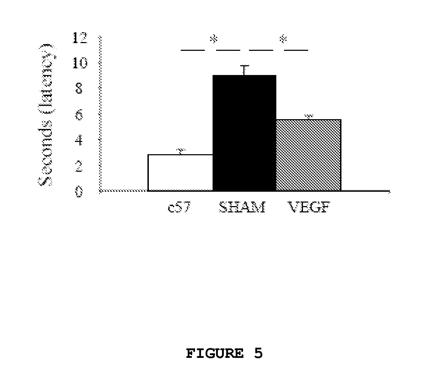

In the T-maze, which measures exploratory activity, there is a recovery in the latency or decision time (Figure 5). The spontaneous alternation rate is also recovered: 63% in the control group, 50% in the mice treated with the microparticles with non-transfected fibroblasts ("sham"), and 61% in the group of mice treated with microparticles with VEGF-producing fibroblasts.

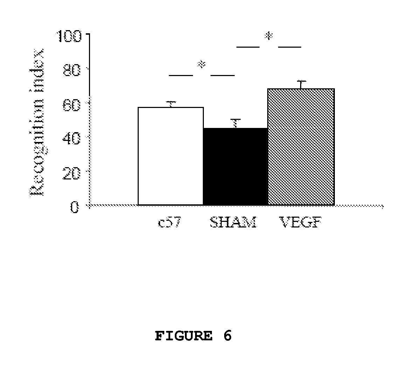

In the object recognition test, which measures short-term memory, there is also a significant recovery (Figure 6).

### EXAMPLE 4

### Treatment of old rats with microparticles in which cells which are genetically modified to produce BDNF have been immobilized

In order to perform this experiment, Wistar rats aged between 24 and 30 months were used, a pilot study with control rats and treated rats being carried out. The cells which are genetically modified to produce BDNF were encapsulated using the same methodology as in Example 1. The microparticles were implanted in the rats by means of a bilateral craniotomy. The control rats were treated in the same manner but empty microparticles were administered to them. The rats remained in the animal housing unit until their sacrifice, at 2 months, to conduct the appropriate analyses.

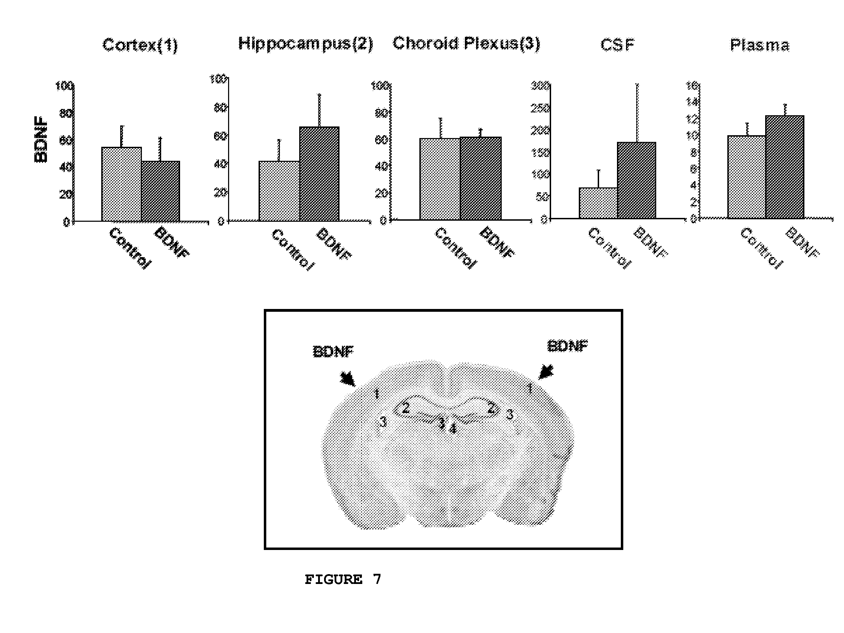

The results which are shown in Figure 7 show that the BDNF released from the microparticles, administered in the surface of the brain, is transported to the blood, following the cortex, hippocampus, choroid plexus, cerebrospinal fluid and blood route.

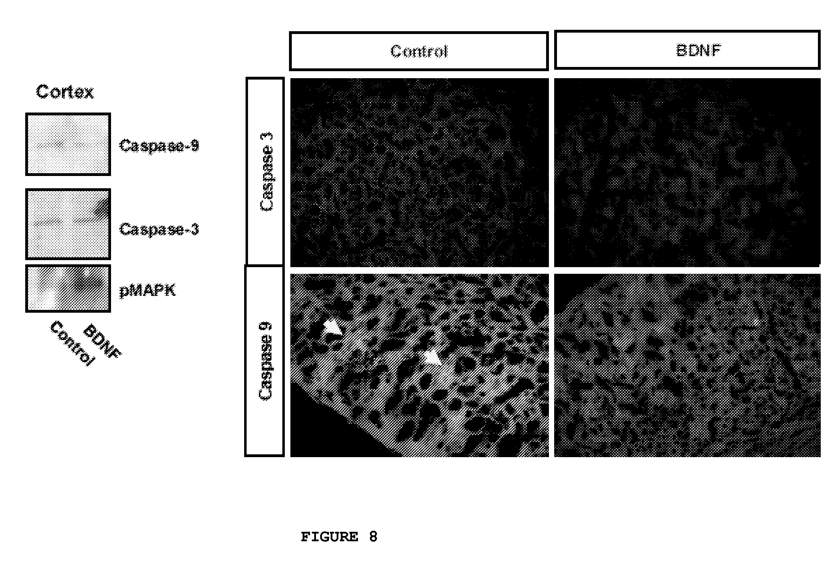

The immunohistochemical analysis for detecting caspase 3, caspase 9 and MAPK in different tissues shows that the rats treated with the BDNF-producing microparticles have, at the cortex level (Figure 8) and at the cerebellum level, a decrease in caspase 3 and caspase 9 activity, which would indicate lower cell death. It can furthermore be observed how an increase of MAPK activity occurs, which indicates the activation of the signaling by BDNF in this region. In the choroid plexuses, it is observed that there are no variations in the levels of BDNF although there is a decrease of the levels of caspase 3, which could indicate a lower deterioration of the blood-brain barrier.

## Claims

1. A microparticle comprising genetically modified cells expressing at least one neurotrophic factor, at least one angiogenic factor or a combination of both for the treatment of neurodegenerative diseases, wherein the microparticle is administered at the cerebral cortex level.

2. The microparticle according to claim 1, wherein the microparticle comprises a polymer bound to a ligand specific for a cell surface receptor.

3. The microparticle according to claim 2, wherein the ligand specific for a cell surface receptor is a peptide comprising the RGD sequence.

4. The microparticle according to claim 2 or 3, wherein the polymer forming part of the microparticle is alginate.

5. The microparticle according to claim 4, wherein the microparticle furthermore comprises a poly-L-lysine membrane.

6. The microparticle according to any of claims 1 to 5, wherein the neurotrophic factor is selected from neurotrophins (NT); brain-derived neurotrophic factor (BDNF); ciliary neurotrophic factor (CNTF); insulin-like growth factor type 1 (IGF-1); insulin-like growth factor type 2 (IGF-2); nerve growth factor (NGF); neurturin (NTN); persephins; artemins, pleiotrophin (PTN), ephrins, netrins, semaphorins, slits, reelins or glial cell-derived neurotrophic factor (GDNF), conserved dopamine neurotrophic factor (CDNF) and mesencephalic astrocyte-derived neurotrophic factor (MANF).

7. The microparticle according to any of claims 1 to 6, wherein the angiogenic factor is selected from angiopoietins (Ang); basic fibroblast growth factor (bFGF/FGF2); transforming growth factor beta (TGFβ), transforming growth factor alpha (TGFα), placental growth factor (PIGF); epidermal growth factor (EGF); vascular endothelial growth factor (VEGF); platelet-derived growth factor (PDGF); vasoactive intestinal polypeptide (VIP); hepatocyte growth factor (HGF), cardiotrophins, bone morphogenetic proteins (BMPs) or sonic hedgehog (SHH).

8. The microparticle according to any of claims 1 to 7, wherein the neurodegenerative disease is selected from Alzheimer's disease, Parkinson's disease, amyotrophic lateral sclerosis and Huntington's disease.

9. The microparticle according to any of claims 1 to 8, wherein the administration of the microparticle at the cerebral cortex level is carried out in a subdural or subarachnoid manner.

10. The microparticle according to any of claims 1 to 9, wherein the administration of the microparticle at the cerebral cortex level is carried out by means of a bilateral craniotomy.


SEQUENCE LISTING

<110> Universidad de País Vasco
Fundación Investigación Biomédica Hospital Universitario 12
Octubre
Centro de Investigación Biomédica en Red de Enfermedades
Neurodegenerativas

<120> USE OF MICROPARTICLES COMPRISING GENETICALLY MODIFIED CELLS IN
THE TREATMENT OF NEURODEGENERATIVE DISEASES

<130> P3694EPPC

<140> EP09800099.5
<141> 2009-07-23

<150> P200802217
<151> 2008-07-24

<160> 34

<170> PatentIn version 3.5

<210> 1
<211> 5
<212> PRT
<213> Artificial

<220>
<223> Peptide derived from the region of the fourteenth type III repeat
of fibronectin

<400> 1

Ile Asp Ala Pro Ser
1               5


<210> 2
<211> 4
<212> PRT
<213> Artificial

<220>
<223> Peptide derived from the fibronectin CS5 region

<400> 2

Arg Glu Asp Val
1


<210> 3
<211> 5
<212> PRT
<213> Artificial

<220>

<223>  Fibrinogen fragment with adhesive capacity

<400>  3

Gln Ala Gly Asp Val
1               5


<210>  4
<211>  4
<212>  PRT
<213>  Artificial

<220>
<223>  Fibronectin fragment with adhesive capacity

<400>  4

Ile Asp Ser Pro
1


<210>  5
<211>  4
<212>  PRT
<213>  Artificial

<220>
<223>  VCAM-I fragment with adhesive capacity

<400>  5

Arg Gly Asp Ser
1


<210>  6
<211>  4
<212>  PRT
<213>  Artificial

<220>
<223>  Peptide derived from the region of the tenth type III repeat of
       fibronectin

<400>  6

Gly Arg Gly Asp
1


<210>  7
<211>  4
<212>  PRT
<213>  Artificial

<220>
<223>  Peptide derived from the region of the tenth type III repeat of

fibronectin

<400>  7

Arg Gly Asp Val
1



<210>  8
<211>  4
<212>  PRT
<213>  Artificial

<220>
<223>  Peptide derived from the region of the tenth type III repeat of
       fibronectin

<400>  8

Arg Gly Asp Thr
1



<210>  9
<211>  5
<212>  PRT
<213>  Artificial

<220>
<223>  Peptide derived from the region of the tenth type III repeat of
       fibronectin

<400>  9

Gly Arg Gly Asp Gly
1               5



<210>  10
<211>  5
<212>  PRT
<213>  Artificial

<220>
<223>  Peptide derived from the region of the tenth type III repeat of
       fibronectin

<400>  10

Gly Arg Gly Asp Ser
1               5



<210>  11
<211>  5
<212>  PRT
<213>  Artificial

<220>
<223>  Peptide derived from the region of the tenth type III repeat of
       fibronectin

<400>  11

Gly Arg Gly Asp Tyr
1               5


<210>  12
<211>  5
<212>  PRT
<213>  Artificial

<220>
<223>  Peptide derived from the region of the tenth type III repeat of
       fibronectin

<400>  12

Gly Arg Gly Asp Phe
1               5


<210>  13
<211>  5
<212>  PRT
<213>  Artificial

<220>
<223>  Peptide derived from the region of the tenth type III repeat of
       fibronectin

<400>  13

Tyr Arg Gly Asp Ser
1               5


<210>  14
<211>  6
<212>  PRT
<213>  Artificial

<220>
<223>  Peptide derived from the region of the tenth type III repeat of
       fibronectin

<400>  14

Tyr Arg Gly Asp Asp Gly
1               5


<210>  15
<211>  6
<212>  PRT

<213>  Artificial

<220>
<223>  Peptide derived from the region of the tenth type III repeat of
      fibronectin

<400>  15

Gly Arg Gly Asp Ser Pro
1               5


<210>  16
<211>  6
<212>  PRT
<213>  Artificial

<220>
<223>  Peptide derived from the region of the tenth type III repeat of
      fibronectin

<400>  16

Gly Arg Gly Asp Ser Gly
1               5


<210>  17
<211>  6
<212>  PRT
<213>  Artificial

<220>
<223>  Peptide derived from the region of the tenth type III repeat of
      fibronectin

<400>  17

Gly Arg Gly Asp Ser Tyr
1               5


<210>  18
<211>  6
<212>  PRT
<213>  Artificial

<220>
<223>  Peptide derived from the region of the tenth type III repeat of
      fibronectin

<400>  18

Gly Arg Gly Asp Val Tyr
1               5


<210>  19

<211>  7
<212>  PRT
<213>  Artificial

<220>
<223>  Peptide derived from the region of the tenth type III repeat of
       fibronectin

<400>  19

Gly Arg Gly Asp Ser Pro Lys
1               5


<210>  20
<211>  8
<212>  PRT
<213>  Artificial

<220>
<223>  Peptide derived from the region of the tenth type III repeat of
       fibronectin

<400>  20

Cys Gly Arg Gly Asp Ser Pro Lys
1               5


<210>  21
<211>  8
<212>  PRT
<213>  Artificial

<220>
<223>  Peptide derived from the region of the tenth type III repeat of
       fibronectin

<400>  21

Cys Gly Arg Gly Asp Ser Pro Lys
1               5


<210>  22
<211>  7
<212>  PRT
<213>  Artificial

<220>
<223>  Peptide derived from the region of the tenth type III repeat of
       fibronectin

<400>  22

Cys Gly Arg Gly Asp Ser Tyr
1               5

<210>  23
<211>  5
<212>  PRT
<213>  Artificial

<220>
<223>  Cyclic peptide derived from the region of the tenth type III
       repeat of fibronectin

<400>  23

Arg Gly Asp Phe Lys
1               5


<210>  24
<211>  8
<212>  PRT
<213>  Artificial

<220>
<223>  Peptide derived from the region of the tenth type III repeat of
       fibronectin

<400>  24

Tyr Ala Val Thr Gly Arg Gly Asp
1               5


<210>  25
<211>  14
<212>  PRT
<213>  Artificial

<220>
<223>  Peptide derived from the region of the tenth type III repeat of
       fibronectin


<220>
<221>  MOD_RES
<222>  (1)..(1)
<223>  Acetylation

<220>
<221>  MOD_RES
<222>  (14)..(14)
<223>  Amidation

<400>  25

Cys Gly Gly Asn Gly Glu Pro Arg Gly Asp Tyr Arg Ala Tyr
1               5                   10


<210>  26

<211>  11
<212>  PRT
<213>  Artificial

<220>
<223>  Peptide derived from the region of the tenth type III repeat of
       fibronectin


<220>
<221>  MOD_RES
<222>  (1)..(1)
<223>  Acetylation

<400>  26

Gly Cys Gly Tyr Gly Arg Gly Asp Ser Pro Gly
1               5                   10


<210>  27
<211>  9
<212>  PRT
<213>  Artificial

<220>
<223>  Peptide derived from the region of the tenth type III repeat of
       fibronectin

<400>  27

Arg Gly Asp Pro Ala Ser Ser Lys Pro
1               5


<210>  28
<211>  4
<212>  PRT
<213>  Artificial

<220>
<223>  Peptide used as spacer element

<400>  28

Gly Gly Gly Gly
1


<210>  29
<211>  4
<212>  PRT
<213>  Artificial

<220>
<223>  Peptide used as spacer element

<400>  29

Gly Gly Gly Ser
1


<210>  30
<211>  13
<212>  PRT
<213>  Artificial

<220>
<223>  Peptide used as spacer element

<400>  30

Gly Gly Gly Gly Gly Gly Gly Gly Gly Gly Gly Gly Gly
1               5                   10


<210>  31
<211>  14
<212>  PRT
<213>  Artificial

<220>
<223>  Peptide used as spacer element

<400>  31

Ser Gly Gly Thr Ser Gly Ser Thr Ser Gly Thr Gly Ser Thr
1               5                   10


<210>  32
<211>  15
<212>  PRT
<213>  Artificial

<220>
<223>  Peptide used as spacer element

<400>  32

Ala Gly Ser Ser Thr Gly Ser Ser Thr Gly Pro Gly Ser Thr Thr
1               5                   10                  15


<210>  33
<211>  7
<212>  PRT
<213>  Artificial

<220>
<223>  Peptide used as spacer element

<400>  33

Gly Gly Ser Gly Gly Ala Pro

1               5

<210>  34
<211>  8
<212>  PRT
<213>  Artificial

<220>
<223>  Peptide used as spacer element

<400>  34

Gly Gly Gly Val Glu Gly Gly Gly
1               5